# EUROPEAN PATENT SPECIFICATION

(11) **EP 1 608 362 B1**
(45) Date of publication and mention of the grant of the patent: **21.02.2007**
(21) Application number: 03770970.6
(22) Date of filing: 10.10.2003
(51) Int. Cl.: A61K 31/40

(54) **STABILIZED PHARMACEUTICAL PREPARATION COMPRISING AN AMORPHOUS ACTIVE SUBSTANCE**
STABILISIERTE PHARMAZEUTISCHE ZUSAMMENSETZUNG ENTHALTEND EINEN AMORPHEN WIRKSTOFF
PREPARATION PHARMACEUTIQUE STABILISEE RENFERMANT UNE SUBSTANCE AMORPHE ACTIVE

(30) Priority: 11.10.2002 SI 200200244
(43) Date of publication of application: 28.12.2005
(73) Proprietor: Lek Pharmaceutical and Chemical Co. D.D., 1526 Ljubljana (SI)
(72) Inventor: BASTARDA, Andrej, 1360 Vrhnika (SI); SALOBIR, Mateja, 1000 Ljubljana (SI); GRAHEK, Rok, 4000 Kranj (SI)
(74) Representative: TBK-Patent
(86) International application number: PCT/EP2003/011265
(87) International publication number: WO 2004/032920

(56) References cited:
- EP-A- 1 241 110
- WO-A1-01/93859
- WO-A1-97/03958
- DE-A1- 2 625 164
- GB-A- 1 376 362
- US-A- 5 682 726
- US-A1- 2002 010 357
- LIEBERMAN H.A.; LACHMAN L. PHARMACEUTICAL DOSAGE FORMS vol. 1, 1980, page 38
- BOCCARDI G. IL FARMACO vol. 49, 1994, pages 431 - 435
- CORNELISSEN P.J.G. PHOTOCHEM AND PHOTOBIOL vol. 30, 1979, pages 337 - 341
- FLORENCE A.T.; ATTWOOD D. PHYSICOCHEMICAL PRINCIPLES OF PHARMACY 2004, pages 103 - 105
- JAVERNIK S. ET AL PHARMAZIE vol. 56, 2001, pages 738 - 740
- KRISTENSEN S. ET AL PHARMAZIE vol. 53, 1998, pages 98 - 103
- MAHAJAN R. ET AL PHARM. RES. vol. 22, 2005, pages 128 - 140
- TONNESEN H.H. PHARMAZIE vol. 54, 1999, pages 590 - 594
- TONNESEN H.H. INT. J. PHARM. vol. 225, 2001, pages 1 - 14
- WATERMAN K.C. PHARM. DEV. TECH. vol. 7, 2002, pages 1 - 32
- WATERMAN K.C.; ADAMI R.C. INT. J. PHARM. vol. 293, 2005, pages 101 - 125

## Description

The present invention belongs to the field of pharmaceutical technology and relates to pharmaceutical preparations comprising amorphous active substances, e.g. amorphous atorvastatin calcium, preferably useful for the treatment of hypercholesterolemia and hyperlipidemia. The invention enables the preparation of a stable pharmaceutical preparation comprising amorphous active substances, known to be unstable in an acidic environment and susceptible to heat, light, moisture and low pH, in a technologically simple way.

Atorvastatin calcium, ((R-(R*,R*))-2(4-fluorophenyl)-β,δ-dihydroxy-5-(1-methylethyl)-3-phenyl-4((phenylamino)carbonyl)-1H-pyrrole-1-heptanoic acid hemi calcium salt) is useful as an inhibitor of 3-hydroxy-3-methylglutaryl-coenzyme A reductase (HMG-CoA reductase), an enzyme involved in the intracellular synthesis of cholesterol. Therefore, HMG-CoA reductase enzyme inhibitors are considered especially useful in the treatment of hypercholesterolemia and hyperlipidemia.

The processes for the preparation of atorvastatin calcium and key intermediates thereof are described in United States Patent Numbers 5,003,080; 5,097,045; 5,103,024; 5,124,482; 5,149,837; 5,155,251; 5,216,174; 5,245,047; 5,248,793; 5,280,126; 5,342,952; and 5,397,792.

Atorvastatin calcium can exist in an amorphous form or in different crystalline forms which are disclosed in the patent applications WO 97/3958; WO 97/3959; WO 01/36384; WO 02/41834; WO 02/43732; WO 02/51804; and WO 02/57229. The processes for the preparation of amorphous atorvastatin calcium are described in the patent applications WO 97/3960; WO 00/71116; WO 01/28999; WO 01/42209; WO 02/57228; and WO 02/59087.

It is well known that active substances in an amorphous form are better soluble and dissolve more rapidly, respectively, than in a crystalline form. The advantage of an amorphous active substance over a crystalline form is particularly evident in case of less soluble substances such as, for example, atorvastatin calcium, having a better bioavailability of the active substance.

It is known from the patent and relevant literature that atorvastatin calcium is an unstable substance which is susceptible to heat, moisture, light and low pH at which atorvastatin calcium is converted from the carboxylic acid form to the lactone form (US 5,686,104; Hurley, T.R. et al. Tetrahedron (1993), 49, 1979-1984). The problem of instability of atorvastatin calcium has been solved thus far by the addition of excipients to a pharmaceutical formulation for the stabilization of atorvastatin calcium, especially in view of the conversion into the lactone form, by addition of a basifying or a buffering agent to a pharmaceutical composition (WO 00/35425; WO 94/16603). A procedure for stabilization of an active substance is known, where in the final phase of synthesis an alkaline substance or a buffering solution is added to prepare an alkaline stabilized substance as described in the patent application WO 01/93860.

The use of a pharmaceutical formulation comprising amorphous atorvastatin calcium as the active substance is advantageous over a pharmaceutical formulation comprising a crystalline substance because the amorphous substance dissolves faster and better which is an important factor for the bioavailability of the active substance in the body. It is well known that the stability of an active substance depends an a polymorphous form in which it exists and that an amorphous form is less stable than a crystalline form indicating that an amorphous form compared to a crystalline form is even more susceptible to heat, light, moisture and low pH. All these factors are of key importance for the stability of a pharmaceutical formulation comprising an amorphous substance. Impurities generated in the degradation of an active substance reduce a therapeutic effect of an active substance and additionally unnecessarily burden the body with unnecessary degradation products. To date an appropriate and useful pharmaceutical preparation containing amorphous atorvastatin calcium has not been described so far.

Therefore, there is a constant need for a stable pharmaceutical formulation comprising amorphous atorvastatin calcium. The object of the present invention is a pharmaceutical preparation comprising amorphous substance atorvastatin calcium having a good bioavailability and being prepared according to a process which is simple and economically convenient, a method of stabilization of the pharmaceutical preparation and the use of this preparation for the manufacture of a medicament in the treatment of hypercholesterolemia and hyperlipidemia. These objects are achieved by the independent claims. Preferred embodiments of the invention are defined in the dependent claims.

A preferred embodiment of the inventive pharmaceutical preparation comprises a pharmaceutical formulation with amorphous atorvastatin calcium exposed to an inert gas atmosphere, wherein the formulation is preferably in a substantially gas exchange non-permeable layer and the inert gas may be contained in the layer. The formulation may be in a substantially gas exchange non-permeable package, preferably selected from the group consisting of an Al/Al blister, an Al-polychloro-3-fluoroethylene homopolymer/PVC laminate blister or a bottle. The inert gas is preferably nitrogen or argon. In the preparation according to the invention the pharmaceutical formulation is prepared in the form of tablets, orally dispersible pharmaceutical formulations, capsules, pellets or granulate. The amorphous active substance is atorvastatin calcium.

In preferred embodiment of a method for packaging a pharmaceutical formulation with amorphous, atorvastatin calcium, a gas exchange non-permeable packaging or layer wherein the packaging procedure is carried out in an inert gas atmosphere defined as above.

In preferred embodiments of methods of stabilization of amorphous atorvastatin calcium or a pharmaceutical formulation comprising amorphous atorvastatin calcium and pharmaceutically acceptable excipients in the pharmaceutical formulation are stored preferably in an inert atmosphere in a package or layer defined as above, wherein the gas exchange non-permeable packaging may be a gas non-permeable plastic bag.

We have surprisingly found that the stability of amorphous atorvastatin calcium is affected by the oxygen content in an environment. There is a linear dependence between the amount of degradation products and the oxygen content in atmosphere. If half of the oxygen content in an atmosphere is replaced with an inert gas under defined temperature conditions, generation of degradation products is halved over a fixed period of time. If amorphous atorvastatin calcium is stored at a defined temperature in an atmosphere with a minimal oxygen content, after a certain period of time the amount of degradation products is less than and/or equal to that when crystalline atorvastatin calcium is stored in air.

According to one embodiment of the present invention there is prepared a stable pharmaceutical preparation comprising as the active substance an amorphous substance known from the literature to be less stable than a crystalline from e.g. amorphous atorvastatin calcium. In this embodiment a pharmaceutical formulation is packaged in a gas exchange non-permeable package or layer in an inert gas atmosphere comprising the active substance. The process for improving the stability of a pharmaceutical preparation comprises storing an amorphous atorvastatin calcium in an inert gas atmosphere during the packaging procedure of the pharmaceutical formulation into a gas exchange non-permeable package or layer such as, for example, Al/Al blister, Al-polychlord-3-fluoroethylene homopolymer/PVC laminate blister and bottles. The process according to the present invention is advantageous over known processes of improving stability of an amorphous active substance because the process is technologically simple and economically non-demanding. A stabilized pharmaceutical preparation according to the present invention comprising e.g. amorphous atorvastatin calcium, does not burden the body with additional substances. An additional advantage of the method for stabilization of the pharmaceutical product of the present invention is that the attained stability of the pharmaceutical preparation is superior to that comprising crystalline atorvastatin calcium.

The pharmaceutical preparation of the present invention comprises a pharmaceutical formulation comprising e.g. amorphous atorvastatin calcium as the active substance and pharmaceutically acceptable excipients. The pharmaceutical formulation may be in any form such as, for example, tablets, orally dispersible pharmaceutical formulations, capsules, pellets, granulate, etc., suitable to be stored in a gas exchange non-permeable package. Preferably nitrogen or argon can be used as inert gas atmosphere in the packaging procedure, wherein nitrogen is especially preferred.

The stable pharmaceutical product of the present invention may be used in the treatment of hypercholesterolemia and hyperlipidemia.

Another aim of the present invention is also to provide a process for preparation of a stable pharmaceutical formulation comprising as an active substance amorphous atorvastatin calcium and pharmaceutically acceptable excipients. This aim is achieved by storing the pharmaceutical formulation in an inert atmosphere thereby achieving the stability which is superior and/or equal to the stability of the pharmaceutical formulation comprising the crystalline active substance. The process of improving the stability of the pharmaceutical formulation comprising amorphous atorvastatin calcium by storing the pharmaceutical formulation in an inert atmosphere is advantageous over known processes of stability improvement of the amorphous active substance, as the process is technologically simple and economically non-demanding. Furthermore, the pharmaceutical formulation, prepared by this process, does not burden the body with additional substances. The term "inert atmosphere" means an atmosphere with a minimal oxygen content.

Analyzing the amount of degradation products in the pharmaceutical formulation comprising amorphous atorvastatin calcium, we have found that the amount of degradation products in the amorphous substance strongly increases when stored in air. We have surprisingly found that the stability of the pharmaceutical formulation is significantly improved if the pharmaceutical formulation is stored in an atmosphere with a minimal oxygen content. If a pharmaceutical formulation comprising amorphous atorvastatin calcium is stored at a defined temperature in an atmosphere with a minimal oxygen content, over a certain period of time the amount of degradation products is less than and/or equal to that in a pharmaceutical formulation comprising crystalline atorvastatin calcium stored in air.

The pharmaceutical formulation of the present invention comprises preferably an amorphous form of atorvastatin calcium as the active substance and pharmaceutically acceptable excipients. The pharmaceutical formulation of the present invention can be any form that is used in pharmaceutical industry such as, for example, tablets, orally dispersible formulations, capsules, pellets, granulate, etc. Nitrogen or argon can be used as the inert gas for maintenance of an inert atmosphere, wherein nitrogen is especially preferred. The pharmaceutical formulation can be stored in an inert atmosphere in Al/Al blister, Al-polychloro-3-fluoroethylene homopolymer/PVC laminate blister or bottles.

The pharmaceutical formulation of the present invention is preferably useful in the treatment of hypercholesterolemia and hyperlipidemia.

A further aim of the present invention is also an improvement of the stability of the active substance amorphous atorvastatin calcium. The aim is achieved by storing the active substance in an inert atmosphere which is considered from the technological and economic point to be an extremely simple solution. Stability of the amorphous active substance stored in this manner is equivalent to the stability of crystalline atorvastatin calcium. The active substance is stored in a practically gas exchange non-permeable package or layer such as, for example, metal containers, glass containers, gas non-permeable plastic bags or containers. Nitrogen or argon can be used as an inert gas for the maintenance of an inert atmosphere, wherein nitrogen is especially preferred.

The stable amorphous active substance of the present invention is useful in the manufacture of a medicament for the treatment of hypercholesterolemia and hyperlipidemia.

The present invention is illustrated but in no way limited by the following examples:

### Example 1

Samples each containing 100 mg of crystalline and amorphous atorvastatin calcium were transferred into 10-ml vials sealed with a rubber-stopper, in an atmosphere with different oxygen content in reference to air composition. Nitrogen of 99% (vol/vol) purity was used as an inert gas. The samples were stored at 80°C for 6 days, and then the amount of impurities was determined by High Performance Liquid Chromatography.
The oxygen content in the vials was determined by gas chromatography with a mass spectrometric detector (GC/MS).

The degradation products were determined by gas chromatography Agilent Technologies (Waldbronn, Germany) model HP 1100. Chromatograms were recorded by a UV detector at 250 nm. The column Chromolit Performance RP-18e 100 x 4.6 mm (Merck, Darmstadt, Germany) and the gradient of mobile phase A: 20 mM ammonium acetate pH 4.0, 5% (v/v) tetrahydrofuran and 25 % (v/v) acetonitrile and the gradient of mobile phase B: 20 mM ammonium acetate pH 4.0, 5% (v/v) tetrahydrofuran and 70 (v/v) acetonitrile were used. The composition of the mobile phase was set that initially and to minute 2 there was 25% of mobile phase B (75% of mobile phase A), from minute 2 to minute 4.7 the composition of the mobile phase changed linearly to 100% of mobile phase B and it remained such to the end of the analysis at minute 5.5. The flow rate of the mobile phase was 7 ml/min. Under the above conditions the retention time of atorvastatin calcium is approximately 1.4 minute. The samples of atorvastatin calcium were dissolved in the solvent 200 mM Tris pH 7, 40% (v/v) acetonitrile of the concentration 0.5 mg/ml.

Analytical method for the determination of the amount of oxygen in the vials and blisters:
The analyses were performed on a gas chromatograph Varian (Walnut Creek, USA) model Varian 3400 and mass detector Finnigan (San Jose, USA) model SSQ700. The capillary column PLOT Coating Molsieve 5A 25m long of internal diameter 0.32 mm (Variana) was used. The temperature of the column was 50°C, of the injector 150°C was and the temperature of the connection with the mass detector was 150°C. The flow through the column was 2 ml of helium per minute. The mode of injection was split with the 1:100 ratio. The mass detector operated by ionization with electrons at 70 eV within the mass region from 20 to 60 mass units in 0.3 second.

Vials and blisters were placed in a chamber with an argon atmosphere. 50 µl gas samples were taken by gas injection and analyzed immediately. The argon atmosphere prevented contamination of the samples with oxygen during sampling.

The signal of the oxygen molecular ion 32 m/z with a retention of approx. 1.9 minutes and the signal of the nitrogen molecular ion 28 m/z with a retention of approx. 3.1 minutes was detected by the gas detector. As a result the oxygen content was calculated in reference to the sum of oxygen and nitrogen.

**Table 1: Increase of the amount of degradation products in the substances stored under stress conditions (6 days at temperature 80°C) in comparison with the starting active substance**

| Oxygen content in the atmosphere in % | Crystalline atorvastatin calcium Increase in the amount of degradation products in % in reference to starting active substance | Amorphous atorvastatin calcium Increase of degradation products in % in reference to starting active substance |
|---|---|---|
| | | |
| 0.6 | 0 | 0 |
| 2.8 | 0 | 0.13 |
| 12.2 | 0.02 | 0.49 |
| 21.3 | 0.05 | 0.89 |

The testing results of the amount of degradation products in crystalline and amorphous atorvastatin calcium at different oxygen contents in atmosphere have demonstrated that at a minimal oxygen content the amount of degradation products is equal in both active substances indicating that the stability of amorphous atorvastatin calcium stored in an inert atmosphere is equal to the stability of crystalline atorvastatin calcium. The measurements also show that the procedure of storing amorphous atorvastatin calcium in an inert atmosphere improves the stability of the amorphous substance but has no influence on the stability of crystalline atorvastatin calcium.

### Example 2

Tablets containing 10 mg of amorphous atorvastatin calcium, which were not previously stabilized by storing in an inert atmosphere, and other pharmaceutically acceptable excipients (microcrystalline cellulose, lactose monohydrate, crosslinked carboxymethyl cellulose, polysorbate 80, hydroxypropyl cellulose, magnesium oxide) were stored in glass 10-ml vials sealed with rubber-stoppers in normal atmosphere (air) and in atmospheres with different oxygen content which was replaced by inert gas. Nitrogen of 99% purity (vol/vol) was used as an inert gas, for comparison, tablets containing 10 mg of crystalline atorvastatin calcium stored in 10-ml rubber-stoppered vials in normal atmosphere (air) were used. Each vial contained one tablet. These vials were placed in a drier for six days under stress conditions at 80°C.

The samples for analysis of the amount of degradation products were prepared by adding 10 ml of a solvent to the tablet in a suitable container and dissolving the tablet by ultrasound in an ultrasound bath for 10 minutes. The tablet disintegrated and the resulting suspension was filtered through the PTFE 0.45 µm injection filter. The clear solution was analyzed by the method disclosed in Example 1.

**Table 2: Increase of the amount of degradation products in comparison with the starting active substance - amorphous atorvastatin calcium in the tablets stored under the stress conditions (6 days at 80°C).**

| Tablet stored in an atmosphere with oxygen content in % | Increase in the amount of degradation products in % in reference to starting active substance |
|---|---|
| | |
| 21.0 | 3.11 |
| | |
| 12.4 | 0.94 |
| | |
| 0.4 | 0.01 |

**Table 3: Increase of the amount of degradation products in comparison with the starting active substance - crystalline atorvastatin calcium in the tablets stored under the stress conditions (6 days at 80°C).**

| Tablet stored in an atmosphere with oxygen content in % | Increase in the amount of degradation products in % in reference to starting active substance |
|---|---|
| | |
| 21.2 | 0.30 |

The test results of the amount of degradation products in the pharmaceutical formulation comprising amorphous atorvastatin calcium stored in atmospheres with different oxygen content have demonstrated that at the minimal oxygen content in an atmosphere, the increase of the amount of degradation products is within the measuring error. The stability of the pharmaceutical formulation comprising amorphous atorvastatin calcium stored in an inert atmosphere, is superior to the stability of a pharmaceutical formulation comprising crystalline atorvastatin calcium, store in a normal atmosphere. On the contrary, when the pharmaceutical formulation comprising amorphous atorvastatin calcium is stored in normal atmosphere, the stability of the active substance is considerably lower because there is an increase of only 3% of the amount of degradation products compared to the initial value of amorphous substance atorvastatin calcium. It is interesting that already after replacing half of the oxygen with an inert gas, the increase drops for two-thirds relative to the values when a pharmaceutical formulation is stored in air.

### Example 3

Tablets containing 20 mg of amorphous atorvastatin calcium, not previously stabilized by storing in an inert atmosphere, and other pharmaceutically acceptable excipients (microcrystalline cellulose, lactose monohydrate, crosslinked carboxymethyl cellulose, polysorbate 80, hydroxypropyl cellulose, magnesium oxide) were packed into blisters with aluminum foil on an industrial blister packaging machine. The first tablet batch was packed in normal atmosphere (air). The second batch, prior to upper foil sealing, packed in an atmosphere of technical argon 99% (v/v). For comparison, the tablets containing 10 mg of crystalline atorvastatin calcium in normal atmosphere were stored in 10-ml vials. The oxygen content in the blister in argon atmosphere was determined by gas chromatography with a mass spectrometric detector (GS/MS).

The stress test of storing the blisters under stress conditions (six days at 80°C) was carried out. For comparison, the tablets containing 10 mg of crystalline atorvastatin calcium in blisters with a normal atmosphere (air) were used and they were exposed to the stress condition. The difference in the amount of impurities of atorvastatin calcium in the blisters was determined by the method described in Example 2.

**Table 4: increase of the amount of degradation products in comparison with the starting active substance - amorphous/crystalline atorvastatin calcium in the tablets depending upon the oxygen content in the atmosphere at packaging**

| | | Amorphous substance | Crystalline substance |
|---|---|---|---|
| Sample | Oxygen content in % in atmosphere | Increase of the amount of degradation products in % to starting active substance | Increase of the amount of degradation products in % to starting active substance |
| | | | |
| Normal atmosphere (air) | | | |
| Tablet 1 | 21.0 | 0.91 | 0.13 |
| Tablet 2 | 21.0 | 0.87 | 0.15 |
| Tablet 3 | 21.0 | 0.95 | 0.12 |
| | | | |
| Inert gas atmosphere | | | |
| Tablet 1 | 2.4 | 0.01 | |
| Tablet 2 | 2.3 | 0.02 | |
| Tablet 3 | 2.1 | 0.03 | |
| | | | |

The test results of the amount of degradation products in the pharmaceutical preparation comprising amorphous atorvastatin calcium when the pharmaceutical formulations were packed into the blisters in an inert gas atmosphere indicate that an increase of the amount of degradation products under stress conditions in the amorphous active substance is within the measuring error suggesting that the amorphous substance, pharmaceutical formulation or pharmaceutical preparation, respectively, comprising the amorphous substance, is stable during prolonged storage if an inert gas atmosphere is used during packaging into blisters. The results surprisingly indicate the fact that the method of stabilization of the pharmaceutical preparation comprising amorphous atorvastatin calcium and comprising packaging of the pharmaceutical formulation into a blister in an inert gas atmosphere provides better results in the stability than the pharmaceutical preparation comprising crystalline atorvastatin calcium packed in air.

## Claims

1. A pharmaceutical preparation comprising a pharmaceutical formulation with amorphous atorvastatin calcium exposed to an inert gas atmosphere.

2. The preparation according to claim 1, wherein the formulation is in a substantially gas exchange non-permeable layer.

3. The preparation according to claim 2, wherein the inert gas is contained in the layer.

4. The preparation according to claim 1, wherein the formulation is in a substantially gas exchange non-permeable package.

5. The preparation according to claim 4 wherein the package is selected from the group consisting of an Al/Al blister, an Al-polychloro-3-fluoroethylene homopolymer/PVC laminate blister or a bottle.

6. The preparation according to any one of the preceding claims, wherein the inert gas is nitrogen or argon.

7. The preparation according to any one of the preceding claims, wherein the pharmaceutical formulation is prepared in the form of tablets, orally dispersible pharmaceutical formulations, capsules, pellets or granulate.

8. A method for packaging a pharmaceutical formulation with amorphous atorvastatin calcium into a gas exchange non-permeable packaging or layer, wherein the packaging procedure is carried out in an inert gas atmosphere.

9. The method according to claim 8, wherein the inert gas atmosphere is contained in the layer.

10. The method according to claim 8, wherein the gas exchange non-permeable packaging is selected from the group consisting of Al/Al blister, Al-polychloro-3-fluoroethylene homopolymer/PVC laminate blister or a bottle.

11. The method according to claims 8 to 10, wherein the inert gas is nitrogen or argon.

12. The method according to claims 8 to 11,
wherein the pharmaceutical formulation is prepared in the form of tablets, orally dispersible pharmaceutical formulations, capsules, pellets or granulate.

13. A method for stabilization of a pharmaceutical formulation comprising amorphous atorvastatin calcium and pharmaceutically acceptable excipients, wherein the pharmaceutical formulation is stored in an inert atmosphere.

14. The method according to claim 13, wherein the gas of the inert atmosphere is nitrogen or argon.

15. The method according to claim 13, wherein the formulation is in a substantially gas exchange non-permeable layer.

16. The method according to claim 15, wherein the inert gas is contained in the layer.

17. The method according to claim 13, wherein the formulation is packed into a gas non-permeable packaging selected from the group consisting of such as Al/Al blister, Al-polychloro-3-fluoroethylene homopolymer/PVC laminate blister or a bottle.

18. The method according to claim 13, wherein the formulation is prepared in the form of tablets, orally dispersible pharmaceutical formulations, capsules, pellets or granulate.

19. A method of stabilization of amorphous atorvastatin calcium, wherein the amorphous atorvastatin calcium is stored in an inert atmosphere.

20. The method according to claim 19, wherein the gas for maintenance of an inert atmosphere is nitrogen or argon.

21. The method according to claim 19, wherein the formulation is in a substantially gas exchange non-permeable layer.

22. The method according to claim 21, wherein the inert gas is contained in the layer.

23. The method according to claim 19, wherein the atorvastatin calcium is packed into a gas non-permeable packaging selected from the group consisting of metal container, glass container, for gas non-permeable plastic bag or for gas non-permeable plastic container.

24. The method according to claim 23, wherein the gas exchange non-permeable packaging is a gas non-permeable plastic bag.

25. The use of the pharmaceutical preparation according to claims 1 to 7 for the manufacture of a medicament in the treatment of hypercholesterolemia and hyperlipidemia.

## Patentansprüche

1. Pharmazeutisches Erzeugnis, das eine pharmazeutische Formulierung mit amorphem Atorvastatin Calcium umfasst, das einer inerten Gasatmosphäre ausgesetzt ist.

2. Erzeugnis nach Anspruch 1, wobei die Formulierung in einer für den Gasaustausch im Wesentlichen undurchlässigen Schicht ist.

3. Erzeugnis nach Anspruch 2, wobei das inerte Gas in der Schicht enthalten ist.

4. Erzeugnis nach Anspruch 1, wobei die Formulierung in einer Verpackung ist, die für den Gasaustausch im Wesentlichen undurchlässig ist.

5. Erzeugnis nach Anspruch 4, wobei die Verpackung ausgewählt ist aus der Gruppe bestehend aus Al/Al-Blister, einem Al-Polychlor-3-fluorethylen-Homopoylmer/PVC-Laminatblister oder einer Flasche.

6. Erzeugnis nach einem der vorhergehenden Ansprüche, wobei das inerte Gas Stickstoff oder Argon ist.

7. Erzeugnis nach einem der vorhergehenden Ansprüche, wobei die pharmazeutische Formulierung in der Form von Tabletten, oral dispergierbaren pharmazeutischen Formulierungen, Kapseln, Perlen oder Granulat zubereitet ist.

8. Verfahren für das Verpacken einer pharmazeutischen Formulierung mit amorphen Atorvastatin Calcium in eine für den Gasaustausch undurchlässige Verpackung oder Schicht, wobei der Verpackungsvorgang in einer inerten Gasatmosphäre durchgeführt wird.

9. Verfahren nach Anspruch 8, wobei die inerte Gasatmosphäre in der Schicht enthalten ist.

10. Verfahren nach Anspruch 8, wobei die für den Gasaustausch undurchlässige Verpackung ausgewählt wird aus der Gruppe bestehend aus Al/Al-Blister, Al-Polychlor-3-fluorethylen-Homopolymer/PVC-Laminatblister oder einer Flasche.

11. Verfahren nach Anspruch 8 bis 10, wobei das inerte Gas Stickstoff oder Argon ist.

12. Verfahren nach Anspruch 8 bis 11, wobei die pharmazeutische Formulierung in der Form von Tabletten, oral dispergierbaren pharmazeutischen Formierungen, Kapseln, Perlen oder Granulat zubereitet wird.

13. Verfahren für die Stabilisierung einer pharmazeutischen Formulierung mit amorphem Atorvastatin Calcium und pharmazeutisch akzeptablen Trägerstoffen, wobei die pharmazeutische Formulierung in einer inerten Atmosphäre gelagert wird.

14. Verfahren nach Anspruch 13, wobei das Gas der inerten Atmosphäre Stickstoff oder Argon ist.

15. Verfahren nach Anspruch 13, wobei die Formulierung in einer für den Gasaustausch im Wesentlichen undurchlässigen Schicht ist.

16. Verfahren nach Anspruch 15, wobei das inerte Gas in der Schicht enthalten ist.

17. Verfahren nach Anspruch 13, wobei die Formulierung in eine für den Gasaustausch undurchlässige Verpackung ausgewählt aus der Gruppe bestehend aus Al/Al-Blister, Al-Polychlor-3-fluorethylen-Homopolymer/PVC-Laminatblister oder einer Flasche verpackt wird.

18. Verfahren nach Anspruch 13, wobei die Formulierung in der Form von Tabletten, oral dispergierbaren pharmazeutischen Formulierungen, Kapseln, Perlen und Granulat zubereitet wird.

19. Verfahren für die Stabilisierung von amorphen Atorvastatin Calcium, wobei das amorphe Atorvastatin Calcium in einer inerten Atmosphäre gelagert wird.

20. Verfahren nach Anspruch 19, wobei das Gas für den Erhalt einer inerten Atmosphäre Stickstoff oder Argon ist.

21. Verfahren nach Anspruch 19, wobei die Formulierung in einer für den Gasaustausch im Wesentlichen undurchlässigen Schicht ist.

22. Verfahren nach Anspruch 21, wobei das inerte Gas in der Schicht enthalten ist.

23. Verfahren nach Anspruch 19, wobei das Atorvastatin Calcium in einer für den Gasaustausch undurchlässigen Verpackung ausgewählt aus der Gruppe bestehend aus einem Metallbehälter, einem Glasbehälter, einem für Gas undurchlässigen Kunststoffbeutel oder einem für Gas undurchlässigen Kunststoffbehälter verpackt wird.

24. Verfahren nach Anspruch 23, wobei die für den Gasaustausch undurchlässige Verpackung ein für Gas undurchlässiger Kunststoffbeutel ist.

25. Verwendung des pharmazeutischen Erzeugnisses nach den Ansprüchen 1 bis 7 für die Herstellung eines Medikaments für die Behandlung von Hypercholesterolämie und Hyperlipidämie.

## Revendications

1. Préparation pharmaceutique comprenant une formulation pharmaceutique avec de l'atorvastatine calcique amorphe exposée à une atmosphère de gaz inerte.

2. Préparation selon la revendication 1, dans laquelle la formulation se situe dans une couche substantiellement imperméable aux échanges gazeux.

3. Préparation selon la revendication 2, dans laquelle le gaz inerte est contenu dans la couche.

4. Préparation selon la revendication 1, dans laquelle la formulation se situe dans un emballage substantiellement imperméable aux échanges gazeux.

5. Préparation selon la revendication 4, dans laquelle l'emballage est choisi dans le groupe constitué par un emballage-coque Al/Al, un emballage laminé homopolymère Al-polychloro-3-fluoroéthylène/PVC et une bouteille.

6. Préparation selon l'une quelconque des revendications précédentes, dans laquelle le gaz inerte est l'azote ou l'argon.

7. Préparation selon l'une quelconque des revendications précédentes, dans laquelle la formulation pharmaceutique est préparée sous la forme de comprimés, de formulations pharmaceutiques dispersibles oralement, de capsules, de pastilles ou de granulés.

8. Procédé de conditionnement d'une formulation pharmaceutique avec de l'atorvastatine calcique amorphe dans un emballage ou une couche imperméable aux échanges gazeux, dans lequel la procédure de conditionnement est réalisée dans une atmosphère de gaz inerte.

9. Procédé selon la revendication 8, dans lequel l'atmosphère de gaz inerte est contenue dans la couche.

10. Procédé selon la revendication 8, dans lequel l'emballage imperméable aux échanges gazeux est choisi dans le groupe constitué par un emballage-coque Al/Al, un emballage laminé homopolymère Al-polychloro-3-fluoroéthylène/PVC et une bouteille.

11. Procédé selon les revendications 8 à 10, dans lequel le gaz inerte est l'azote ou l'argon.

12. Procédé selon les revendications 8 à 11, dans lequel la formulation pharmaceutique est préparée sous la forme de comprimés, de formulations pharmaceutiques dispersibles oralement, de capsules, de pastilles ou de granulés.

13. Procédé de stabilisation d'une formulation pharmaceutique comprenant de l'atorvastatine calcique amorphe et des excipients pharmaceutiquement acceptables, dans lequel la formulation pharmaceutique est conservée dans une atmosphère inerte.

14. Procédé selon la revendication 13, dans lequel le gaz de l'atmosphère inerte est l'azote ou l'argon.

15. Procédé selon la revendication 13, dans lequel la formulation se situe dans une couche substantiellement imperméable aux échanges gazeux.

16. Procédé selon la revendication 15, dans lequel le gaz inerte est contenu dans la couche.

17. Procédé selon la revendication 13, dans lequel la formulation est conditionnée dans un emballage imperméable aux gaz choisi dans le groupe constitué par un emballage-coque Al/Al, un emballage laminé homopolymère Al-polychloro-3-fluoroéthylène/PVC et une bouteille.

18. Procédé selon la revendication 13, dans lequel la formulation est préparée sous la forme de comprimés, de formulations pharmaceutiques dispersibles oralement, de capsules, de pastilles ou de granulés.

19. Procédé de stabilisation d'atorvastatine calcique amorphe, dans lequel l'atorvastatine calcique amorphe est conservée dans une atmosphère inerte.

20. Procédé selon la revendication 19, dans lequel le gaz pour maintien d'une atmosphère inerte est l'azote ou l'argon.

21. Procédé selon la revendication 19, dans lequel la formulation se situe dans une couche substantiellement imperméable aux échanges gazeux.

22. Procédé selon la revendication 21, dans lequel le gaz inerte est contenu dans la couche.

23. Procédé selon la revendication 19, dans lequel l'atorvastatine calcique est conditionnée dans un emballage imperméable aux gaz choisi dans le groupe constitué par un récipient métallique, un récipient en verre, un sac en plastique imperméable aux gaz et un récipient en plastique imperméable aux gaz.

24. Procédé selon la revendication 23, dans lequel l'emballage imperméable aux échanges gazeux est un sac en plastique imperméable aux gaz.

25. Utilisation de la préparation pharmaceutique selon les revendications 1 à 7 pour la fabrication d'un médicament destiné au traitement de l'hypercholestérolémie et de l'hyperlipidémie.
